# EUROPEAN PATENT APPLICATION

(11) **EP 1 764 409 A1**
(43) Date of publication of application: **21.03.2007**
(21) Application number: 05291877.8
(22) Date of filing: 12.09.2005
(51) Int. Cl.: C12M 1/34

(54) **Device and method for observing the organization of studied cells cultured in the presence of a concentration gradient of chemotactic molecules**

(71) Applicant: INSTITUT NATIONAL DE LA SANTE ET DE LA RECHERCHE MEDICALE (INSERM), 75013 Paris (FR)
(72) Inventor: Savagner, Pierre, 34790 Grabels (FR); Muller, Ines, 69126 Heidelberg (DE)
(74) Representative: Blot, Philippe Robert Emile

(57) **Abstract**

The device comprises a cell culture compartment (10) and a diffusion compartment (20) in fluid communication with the cell culture compartment (20) through a filtering element (22) for the progressive diffusion of chemotactic molecules contained in the diffusion compartment into a culture medium contained in the culture compartment, so as to maintain, in the culture compartment, a chemotactic molecules concentration gradient between a region (A) of the culture compartment (10) adjacent to the diffusion compartment (20) and a region (B) of the culture compartment (10) spaced from the diffusion compartment (20), wherein the ratio (R) of the volume of the culture compartment (10) usable for receiving a culture medium over the volume of the diffusion compartment (20) usable for receiving molecules is at least 15.

## Description

The present invention relates to a device for observing the reaction of cells cultured in the presence of a concentration gradient of chemotactic molecules.

Such devices called Boyden chamber and Zigmond chamber are known.

The Boyden chamber comprises two superimposed compartments separated by a microporous membrane. A culture medium containing the cells is placed in the upper compartment and a chemoattractant solution containing the chemotactic molecules is placed in the lower compartment.

During the test, a concentration gradient of chemotactic molecules develops across the thickness of the membrane. The cells subjected to said gradient migrate through the membrane more or less rapidly depending on the chemotactic molecules.

The cell movement into the membrane is measured after an incubation period less than the period taken for the gradient to decay.

The Zigmond chamber comprises two compartements and a bridge extending between said compartments. A culture medium is placed over the bridge, a chemoattractant solution containing the chemotactic molecules is placed in one of the compartments, and cells are placed in the other compartment.

If there is chemotactic activity, the cells will migrate over the bridge towards the compartment containing the chemoattractant solution.

These devices are adapted to observe the migration of the cells so as to determine if there is a chemotactic activity in presence of given type of chemotactic molecules.

Nevertheless, these devices are not adapted for observing the reaction of tissue cells subjected to the concentration gradient of chemotactic molecules, namely the organisation of the tissue cells, i.e. the development of forms and structures between the tissue cells, subjected to the concentration gradient of chemotactic molecules.

Tissue cells are cells of the same type having the same function and adapted to form in a living body a tissue analogous e.g. to an interlacing or to fibres, such as muscular, nervous, epithelial or connective tissue.

Yet, concentration gradient of chemotactic molecules is believed to be of great importance in the differentiation of cells, i.e. the act or process of acquiring completely individual characters, such as occurs in the progressive diversification of tissue cells of the embryo, and the study of the reaction of tissue cells is important namely in the cancer research field.

Therefore, there is a need for a device for observing the organization of cells subjected to a concentration gradient of chemotactic molecules.

To this end, the invention proposes a device for observing the organization of studied cells cultured in the presence of a concentration gradient of chemotactic molecules which device comprises a cell culture compartment defined in a container and a diffusion compartment in fluid communication with the cell culture compartment via a passage through a filtering element for the progressive diffusion of chemotactic molecules contained in the diffusion compartment into a culture medium contained in the culture compartment, so as to maintain, in the culture compartment, a chemotactic molecules concentration gradient between a region of the culture compartment adjacent to an outlet of the passage emerging in the culture compartment and a region of the culture compartment spaced from the outlet, wherein the ratio of the volume of the culture compartment usable for receiving a culture medium over the volume of the diffusion compartment usable for receiving molecules is at least.

Different embodiments of the device for observing the organization of studied cells may comprise any of the following features, individually or in any technically feasible combination :
- the filtering element is a microporous membrane,
- pores of the microporous membrane have a diameter less than 0.3 micrometers,
- an insert affixed to the container in the culture compartment, the insert delimiting the diffusion compartment,
- the insert comprises a tubular body laterally delimiting the diffusion compartment, and wherein the microporous membrane extends across an open end of the tubular body which is affixed to the bottom of the container via a spacer for maintaining the microporous membrane spaced from the bottom of the container,
- the container has a transparent bottom, whereby the organization of cells is observable through said bottom.

The invention also relates to a method for observing the organization of studied cells subjected to a molecule concentration gradient, wherein cells are cultured in a device as defined above and molecules are diffused from the diffusion compartment, thereby forming a concentration gradient towards the studied cells.

The method for observing the organization of studied cells may comprise any of the following features, individually or in any technical and feasible combination:
- chemotactic molecules are provided into the diffusion compartment,
- the molecules are provided in the form of a sponge or a pharmaceutical capsule that gradually releases the molecules,
- chemotactic molecules releasing cells are provided into the diffusion compartment,
- the step of providing in the culture compartment wherein a substrate adhered to a wall of the culture compartment and adapted for the studied cells to anchor onto said substrate, the studied cells being deposited in the substrate,
- the substrate is specific to said studied cells.

The invention further relates to a kit for preparing a device as defined above, comprising an resert, a microporous membrane, and a spacer.

The invention will be better understood on reading the following description which is given by way of example only and with reference to the appended drawings, in which:
- Figure 1 is a perspective exploded view of a device according to the invention-;
- Figure 2 is a lateral cross section view of the assembled device of Figure 1; and
- Figure 3 is a top view of the assembled device of Figure 1.

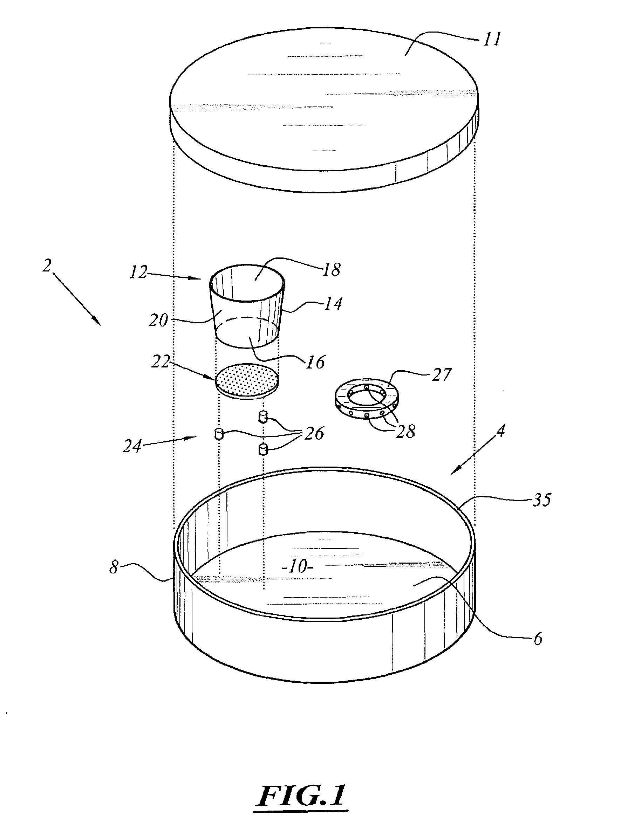

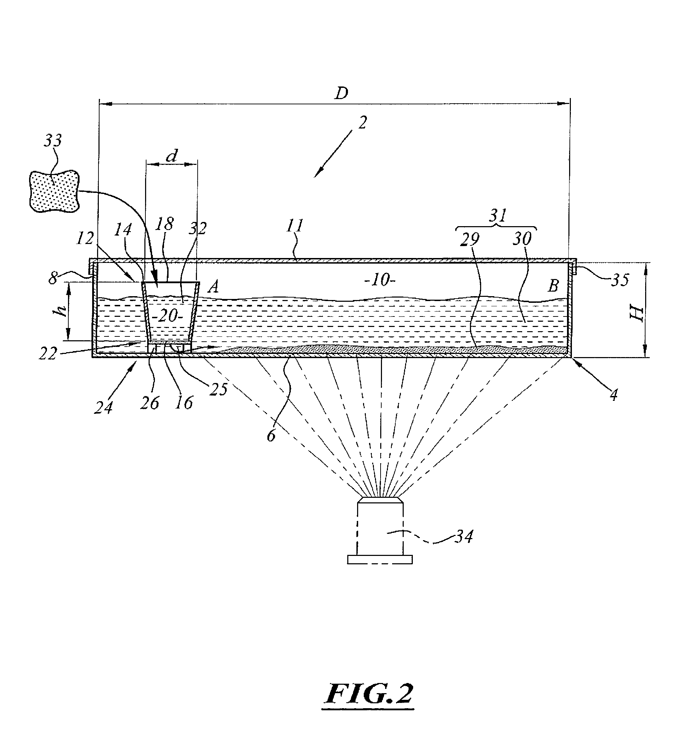

With reference to Figure 1 and 2, the device 2 comprises a container 4 having a bottom 6 and lateral walls 8 extending upwards at the periphery of the bottom 6, said bottom 6 and said walls 8 delimiting a cell culture compartment 10, with an upper open end.

The container 4 is for example circular in shape, and may have any other shape, such as a rectangular shape. The container 4 is for example a Petri dish which is widely used for cell culture.

The bottom 6 is preferably transparent whereby the compartment 10 can be observed or lightened through said bottom 6. The container 4 is for example a Petri dish made of a transparent material, such as glass, for example Pirex^{®}.

The device 2 comprises a cover 11 adapted for closing the upper open end of the container 4.

The device 2 comprises a well 12 having a hollow tubular body 14 with a lower open end 16, an upper open end 18, and a lumen 20 extending between ends 16 and 18.

The well 12 is for example cylindrical and may have a section of any other shape. The well 12 is made for example of plastic, glass, namely Pyrex^{®} or metal.

The device 2 comprises a microporous membrane 22 closing the lower end 16. The membrane 22 has pores of a diameter of for example less than 0.5 micrometer, preferably less than 0.3 micrometer. The membrane 22 is made for example of polycarbonate.

The end 16 is affixed to the bottom 6 via a spacer 24 maintaining the membrane 22 spaced from the bottom 6. The spacer 24 is for example adhered to the bottom 6 and to the end 16.

A passage 25 (Figure 2) is created under the membrane 22, namely between the membrane 22 and the bottom 6.

The passage 25 is in fluid communication with the compartment 10. To this end, the spacer 24 comprises for example three independent spaced-apart feet 26 (Figure 1) angularly regularly spaced around the axis of the well 12, whereby the passage 25 is in communication with the chamber 10 between the feet 26.

The lumen 20 defines a diffusion compartment (Figure 2) within compartment 10, in fluid communication with compartment 10 through the membrane 22 and the passage 25. The diffusion compartment is hereinafter referenced to as the compartment 20.

The well 12 is off-centered with respect to container 4.

The compartment 20 has a volume usable for receiving a chemoattractant solution containing chemotactic molecules that is less that the volume of the compartment 10 usable for receiving a culture medium containing studied cells. The ratio R of the volume of compartment 10 over the volume of compartment 10 is preferably at least 15, more preferably at least 20. This ratio is preferably less than 200.

To this end, the diameter d of the well 12 is for example 5 to 10 times less than the diameter D of the container 4.

The diameter D is for example comprised between 35 to 100 mm, which are diameters of conventional Petri dishes, and the diameter d is for example of between 3 to 10 mm.

In one example, the diameter d is 5 mm and the diameter D is 35 mm, which is the diameter of conventional Petri dishes of small size, and the respective heights H, h of compartments 10 and 20 are substantially the same. In this case, the volume of compartment 10 substantially equals H·π·D²/4, the volume of compartment 20 substantially equals h·π·d²/4, and the ratio R is approximately 49.

In an alternative, the feet spacing the membrane 22 from the bottom 6 are replaced by a ring 27 mating the end 16 interposed between the bottom 6 and the end 16, said ring having radial holes 28 for fluid communication between inside and outside the ring 27.

In an alternative, the well 12 is affixed on a lateral wall 8, the end 16 being spaced from the bottom 6. In this case, the membrane 22 is maintained spaced from the bottom 6 by the wall 8 and it is not necessary to provide a specific spacer.

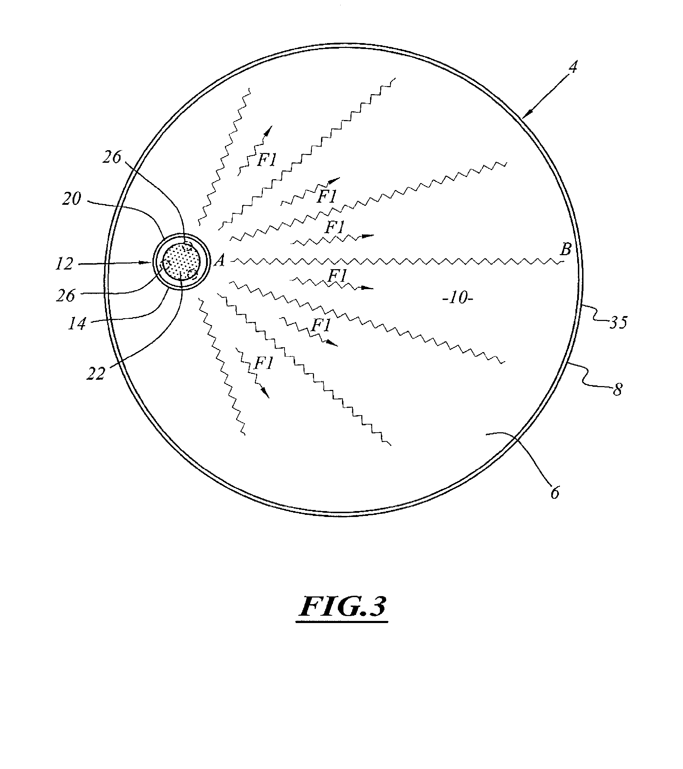

A method for observing the organization of tissue of cells will now be described with reference to Figure 2 and 3.

In a first step, a substrate 29 is placed over the bottom 6. The substrate 29 adheres to the bottom 6 and provides an anchoring structure for the tissue cells, said anchoring structure being analogue to the structure of tissues of the body of an animal or a human.

The substrate 29 is for example an organic substrate comprising inactivated living substrate tissue cells or dead substrate tissue cells, such as animal or human cells. Said inactivated or dead substrate cells have no activity that could interfere with the activity of the studied cells subjected to a concentration gradient of chemotactic molecules.

Such an organic substrate is obtained for example by culturing the alive substrate cells in the container 4, whereby the substrate cells can adhere to the bottom 6 thanks to natural substances secreted by the substrate cells, until the quantity of substrate 29 is sufficient, and killing or inactivating the substrate cells in a known manner, for example by exposure to UV radiations or gamma irradiations.

Preferably, the substrate 29 is composed of substrate tissue cells that are specific of the cells to study, i.e. cells of tissues with which the cells to study are in contact in the animal or human body.

In an alternative, the substrate cells are maintained alive and active, in order to study the reaction of the studied cells in presence of the substrate cells. The active substrate cells might as a matter of fact have an influence on the organization of the studied cells, and also react to the concentration gradient.

In a second step, the studied cells are introduced in the substrate 29. In an alternative, the studied cells are introduced in the substrate 29 before placing the substrate 29 in the compartment 10.

In a third step, the substrate 29 is covered with a culture medium 30. As it is known, the culture medium 30 contains substances that are adapted to maintain the studied cells, and optionally the substrate cells, alive.

The substrate 29 and the culture medium 30 form together a culture means 31.

A chemotactic molecules releasing medium 32 containing chemotactic molecules is placed in compartment 20.

As it is known, said releasing medium 32 is for example a culture medium containing releasing cells, such as cells known under the "3T3 cells" denomination, said releasing cells containing the chemotactic molecules and progressively releasing these molecules. In an alternative, the releasing medium containing the chemotactic molecules is a gel, a sponge 33 (Figure 2), or a releasing device such as a pharmaceutical capsule made of an aggregate of hollow globules adapted for progressively opening and releasing molecules contained therein.

Preferably, the container 4 is sealingly closed with the cover 11, to maintain a sterile atmosphere in the compartment 10.

Due to the higher concentration of chemotactic molecules in compartment 20 with respect to compartment 10, the chemotactic molecules progressively diffuse from compartment 20 to compartment 10 through the membrane 22 and the passage 25.

The pores of membrane 22 have a diameter that is too small for the cells to culture or the 3T3 cells to pass therethrough but which is large enough for the chemotactic molecules to pass therethrough.

The chemotactic molecules diffuse out of the passage 25 radially from the well 12 (Arrows F1). Therefore, a molecule concentration gradient is formed in compartment 10 and is a directed from a region A of higher concentration around well 12, toward a region B of lower concentration at the periphery of compartment 10, more particularly at part 35 of the periphery of container 10 that is the most distant from well 12.

The concentration gradient forms through the culture medium 30 and through the substrate 29. The cells are thus subjected to the concentration gradient in the substrate 29 and at the interface between the substrate 29 and the culture medium 30.

The studied cells will thus differentiate in the presence of the concentration gradient, and organize themselves in forms and structures.

The studied cells can be observed from above. However, the studied cells are thus observed through culture medium 30.

Preferably, the studied cells are observed from below through bottom 6, as illustrated on Figure 2, using a microscope head 34.

In one example, the cells are human mammary epithelial cells, and the substrate 29 is made of inactivated human mammary fibroblast cells. In another example, the substrate 29 is made of collagen.

For observing the organization of studied tissue cells subjected to a concentration gradient of chemotactic molecules, it is important to subject the cells to the concentration gradient over a long period of time.

According to one aspect of the invention, due to the difference of volume between compartment 10 and compartment 20, the molecule gradient can be maintained over a long period of time since it takes a long time for the chemotactic molecules to diffuse in the culture medium 30.

Although the substrate 29 and the culture medium 30 do not entirely fill the volume of compartment 10, the ratio R' of the volume of the substrate 29 and the culture medium 30 over the volume of the releasing medium 32 is still sufficient for maintaining a long term concentration gradient.

The ratio R' is preferably at least 5, more preferably at least 10.

This is the case in a Petri dish such as container 4, since such dishes have a height of substantially 10 mm, and are filled up to 3 mm, and more preferably between 5 and 8 mm.

The device of the invention helps carrying out the method with the ratio R', since if the user fills up the compartments 10 and 20, the R'-ratio conditions will be fulfilled. It is possible to carry out the method of the invention in a device which does not fulfil the R-ratio condition, provided the R'-ratio condition is fulfilled.

The kinetics of the diffusion of the chemotactic molecules depends upon the area of the exchange surface between compartments 10 and 20.

In order to be able to adjust the kinetics, the exchange surface is preferably comprised between 0,1 mm² and 20 mm².

In the embodiment of Figure 1-3, passage 25 extends between compartments 20 and 10. The exchange surface depends, at the end of passage 25 adjacent compartment 20, upon the size of the pores of the membrane 22, the area of the membrane 22 itself, and the density of the pores on the membrane 22.

At the end of passage 25 adjacent compartment 10, the exchange surface is dependent upon the section of the outlet of passage 25 into compartment 10, whereby the space between the feet 26 (figure 1), or the diameter and the number of holes 28 (figure 1) enable to modify the area of the exchange surface.

The membrane 22 contribute to slow down the diffusion of the chemotactic molecules, and to maintain the molecule gradient over a longer period of time.

According to another aspect of the invention, the cells are anchored in the substrate 29. Consequently, it is possible to remove the culture medium 30 in the course of the test without removing the cells, and without modifying the structure of the cells, and to renew the culture medium 30, thus keeping the cells alive and active a longer time. Moreover, a new concentration gradient of chemotactic molecules is formed in the new culture medium 30, whereby the cells are subjected to a concentration gradient over a longer period of time. It is possible to renew the culture medium using a peristaltic pump.

This feature of the invention contributes to solve the problem of subjecting the studied cells to a concentration gradient of chemotactic molecules over a long period of time with the feature related to the ratio R. This feature is also usable independently of the feature related to the ratios R and R'.

Accordingly, a method of observing the reaction of cells subjected to a concentration gradient of molecules comprises the steps of placing a substrate into the compartment, said substrate being adapted for the cells to anchor onto said substrate, introducing the cells in said substrate, covering the substrate with a culture solution, and subjecting the cells to a concentration gradient of chemotactic molecules by forming a concentration gradient of chemotactic molecules across the compartment.

Moreover, the substrate constitutes a base for the cells to develop their forms and structures, and in the case the substrate is specific to the cells, the substrate enables to better reproduce the normal conditions encountered by the cells in the natural environment, e.g. in an animal or human body. The results of the test are thus more reliable.

To maintain the concentration gradient, it is also possible to fill again the diffusion compartment 20 so as to provide more chemotactic molecules, particularly in the case the culture solution has been renewed.

It is possible to provide a kit comprising the well 12, the membrane 22 and the spacer 24 so as to enable a user to obtain the device of the invention, using for example a Petri dish as a main container.

## Claims

1. A device for observing the organization of studied cells cultured in the presence of a concentration gradient of chemotactic molecules, which device comprises a cell culture compartment (10) defined in a container (4) and a diffusion compartment (20) in fluid communication with the cell culture compartment via a passage (25) through a filtering element (22) for the progressive diffusion of chemotactic molecules contained in the diffusion compartment (20) into a culture medium contained in the culture compartment (10), so as to maintain, in the culture compartment, a chemotactic molecules concentration gradient between a region (A) of the culture compartment (10) adjacent to an outlet of the passage (25) emerging in the culture compartment (10) and a region (B) of the culture compartment (10) spaced from the outlet, wherein the ratio (R) of the volume of the culture compartment (10) usable for receiving a culture medium over the volume of the diffusion compartment usable for receiving molecules is at least 15.

2. The device according to claim 1, wherein the filtering element is a microporous membrane (22).

3. The device according to claim 2, wherein pores of the microporous membrane (22) have a diameter less than 0.3 micrometers.

4. The device according to claim 2 or 3, comprising an insert (14) affixed to the container in the culture compartment, the insert delimiting the diffusion compartment (20).

5. The device according to claim 4, wherein the insert comprises a tubular body (14) laterally delimiting the diffusion compartment (20), and wherein the microporous membrane (22) extends across an open end (16) of the tubular body (14) which is affixed to the bottom (6) of the container (4) via a spacer (24) for maintaining the microporous membrane (22) spaced from the bottom (6) of the container (4).

6. Device according to any of claims 1 to 5, wherein the container has a transparent bottom (6), whereby the organisation of cells is observable through said bottom (6).

7. A method for observing the organization of studied tissue cells subjected to a molecule concentration gradient, wherein cells are cultured in the device defined according to any of claims 1 to 6, and molecules are diffused from the diffusion compartment (20), thereby forming a concentration gradient towards the studied cells.

8. The method of claim 7, wherein chemotactic molecules are provided into the diffusion compartment (20).

9. The method of claim 8, wherein the molecules are provided in the form of a sponge or a pharmaceutical capsule that gradually releases the molecules.

10. The method of claim 7, wherein chemotactic molecules releasing cells are provided into the diffusion compartment.

11. The method of any of claims 7 to 10, comprising the step of providing in the culture compartment (10) a substrate (29) adhered to a wall (6) of the culture compartment (10) and adapted for the studied cells to anchor onto said substrate (29), the studied cells being deposited in the substrate (29).

12. The method of claims 11, wherein the substrate is specific to said studied cells.

13. Kit for preparing a device according to claim 5, comprising an insert, a microporous membrane (22), and a spacer.
